# EUROPEAN PATENT APPLICATION

(11) **EP 3 002 013 A1**
(43) Date of publication of application: **06.04.2016**
(21) Application number: 14382379.7
(22) Date of filing: 02.10.2014
(51) Int. Cl.: A61L 9/20, A61L 9/22, F24F 3/16

(54) **Air sterilizing unit**

(71) Applicant: Aero Engineering, S.L., 08173 San Cugat del Valles (ES)
(72) Inventor: Becerril Ruiz, Jose Luis, 08198 SANT CUGAT DEL VALLES (ES); Hernandez Diaz, David, 43007 TARRAGONA (ES); Fernandez Martinez, Albert, 08012 BARCELONA (ES)
(74) Representative: Igartua, Ismael

(57) **Abstract**

Air sterilizing unit comprising ion generating means (2) in which air for photocatalytic oxidation is prepared, and a photocatalytic oxidation chamber (3) in which photocatalysis takes place. The photocatalytic oxidation chamber (3) comprises at least one surface of activation (4) coated with titanium dioxide nanofibers and arranged on a base (6), and at least one UV LED (5) irradiating light on said surface of activation (4), the shape of said surface of activation (4) being configured according to the radiation pattern of the LED (5).

## Description

### TECHNICAL FIELD

The present invention relates to air sterilizing units.

### PRIOR ART

Titanium dioxide is known to have photocatalytic capacity when it receives UV radiation at specific wavelengths, i.e., the capacity to create positive and negative electric charges that can break water molecules down into hydroxyl radicals, which in turn can break organic molecules and destroy them.

In addition, use of photocatalytic oxidation chambers in air sterilizing units is known. Document ES2398765A1, for example, relates to a sterilizing unit intended for being interposed in an air conduit. The sterilizing unit comprises successively turbulent flow generating means, a negative ion generator, an ozone generator, a chamber for the ozone and negative ions to react with air and an ozone neutralizer. The sterilizing unit further comprises a first additional photocatalytic oxidation chamber comprising an air inlet, a negative ion generator, a section coated with titanium dioxide in which a plurality of UV LEDs are arranged, and an air outlet.

### DISCLOSURE OF THE INVENTION

The object of the invention is to provide an air sterilizing unit as described below.

The air sterilizing unit of the invention comprises ion generating means in which air for photocatalytic oxidation is prepared and a photocatalytic oxidation chamber in which photocatalysis takes place.

The photocatalytic oxidation chamber comprises at least one surface of activation coated with titanium dioxide nanofibers arranged on a base and at least one UV LED (Light Emitting Diode) irradiating light on said surface of activation.

The shape of said surface of activation is configured according to the radiation pattern of the LED, which allows maximizing the surface of incidence of the UV light.

Using UV LEDs instead of conventional UV lamps reduces power consumption of the sterilizing unit. Furthermore, it is cleaner technology because contaminating materials are not used during its manufacture and its durability is substantially greater than that of conventional UV lamps.

The drawback of using UV LEDs is that the surface of irradiation and illumination that can be covered by the LEDs is limited, so it is necessary to increase the relative surface area of activation of titanium dioxide without increasing the actual surface area of illumination. To that end, titanium dioxide nanofibers are used as they increase the active surface area without increasing the actual surface area of illumination.

Furthermore, since UV LEDs are smaller than UV lamps and titanium dioxide nanofibers are used to increase the relative surface area of activation of the titanium dioxide without increasing the actual surface area of illumination, they allow obtaining more compact photocatalytic oxidation chambers. On the other hand, as indicated it is possible to maximize the actual surface area of illumination because the active surface is configured taking into account the radiation pattern of the LEDs.

These and other advantages and features of the invention will become evident in view of the drawings and the detailed description of the invention.

### DESCRIPTION OF THE DRAWINGS

Figure 1 shows a schematic view of a first embodiment of the sterilizing unit according to the invention.
Figure 2 shows a partial section view of the photocatalytic oxidation chamber of the sterilizing unit of Figure 1.
Figure 3 shows a front view of the photocatalytic oxidation chamber of the sterilizing unit of Figure 1.
Figure 4 shows a schematic view of a second embodiment of the sterilizing unit according to the invention.
Figure 5 shows a schematic view of a third embodiment of the sterilizing unit according to the invention.

### DETAILED DISCLOSURE OF THE INVENTION

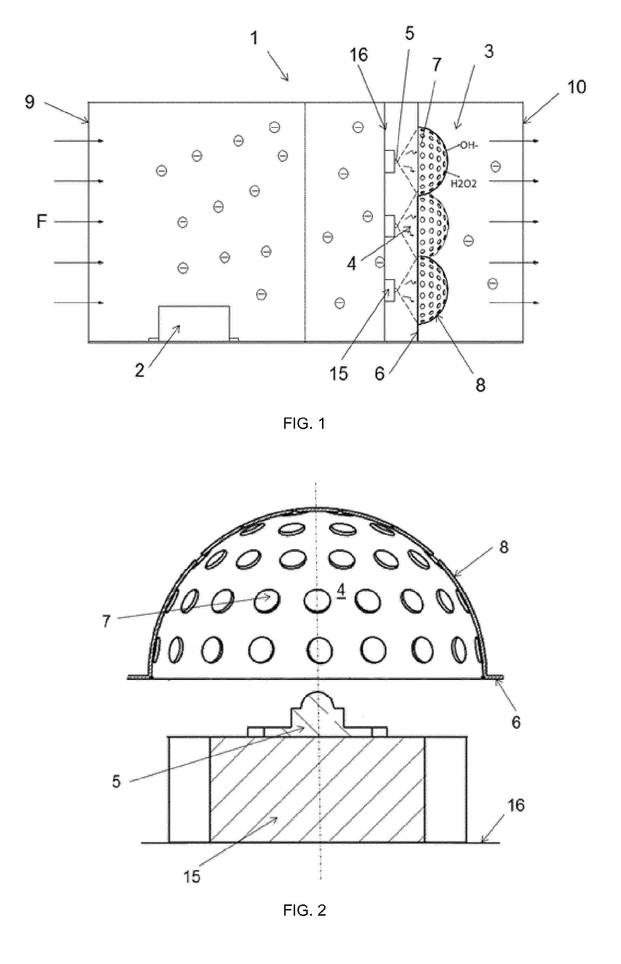

Figure 1 shows a first embodiment of the air sterilizing unit 1 according to the invention.

The air sterilizing unit 1 comprises ion generating means 2 in which air for photocatalytic oxidation is prepared, and a photocatalytic oxidation chamber 3 in which photocatalysis takes place.

The sterilizing unit 1 also comprises an inlet 9 through which the air to be sterilized is introduced and an outlet 10 through which the air exits after sterilization.

The photocatalytic oxidation chamber 3 comprises a plurality of surfaces of activation 4 coated with titanium dioxide nanofibers. Said surfaces of activation 4 are arranged on a base 6. In this embodiment, the surfaces coated to form the surfaces of activation 4 are metallic.

The photocatalytic oxidation chamber 3 further comprises a plurality of UV LEDs 5 irradiating light on said surfaces of activation 4.

The shape of the surfaces of activation 4 is configured such that the surface of incidence of UV light is maximized, being adapted to the radiation pattern of the LED 5.

Using UV LEDs 5 instead of conventional UV lamps reduces power consumption of the sterilizing unit 1. Furthermore, it is cleaner technology because contaminating materials are not used during manufacture. Generating UV light by means of LEDs 5 instead of conventional lamps allows having different light emitting sources without escalating power consumption. The air going through the photocatalytic oxidation chamber 3 is therefore irradiated by UV light for a longer time, thus improving the process.

The drawback of using UV LEDs 5 instead of conventional UV lamps is that the surface of irradiation and illumination that can be covered by the LEDs 5 is limited, so it is necessary to increase the relative surface area of activation of titanium dioxide without increasing the actual surface area of illumination. To that end, titanium dioxide nanofibers are used as they increase the active surface area without increasing the actual surface area of illumination.

In this first embodiment, the sterilizing unit 1 comprises a plurality of domes 8 with holes 7. Each of said domes has a LED 5 associated therewith which is arranged facing the inner surface of the dome, the inner surface of said dome 8 corresponding with the surface of activation 4.

Optionally, the outer surface of the domes 8 can be coated with titanium dioxide nanofibers.

In this embodiment, the domes 8 are half-sphere shaped, the base of the dome 8 being circular. In other possible embodiments, the dome can have a square, polygonal or elliptical base.

In this embodiment, the LED 5 associated with each of the domes 8 is arranged in the axis of symmetry of the dome, as can be seen in Figure 2. In other possible embodiments, each dome 8 can have more than one UV LED 5 associated therewith. The holes 7 of the domes 8 allow the UV light to be irradiated out of the dome as well.

In this embodiment, each LED 5 is arranged on a heat sink 15. Said sinks 15 are arranged on a second base 16 parallel to the base 6 on which the domes 8 are arranged.

The arrangement of LED 5 with respect to the center of the base of the dome 8 depends on the power, angle of emission and wavelength of the LED 5. The maximum distance that may exist between the LED 5 and the point of the dome 8 farthest from said LED 5 is defined according to said parameters to obtain the minimum energy capable of activating the surface of activation 4.

Optionally, a collimating lens (not shown in the drawings) can be arranged inside each dome 8 to homogenize the radiation striking the surfaces of activation 4.

In this embodiment, the base 6 on which there are arranged domes 8 is arranged perpendicular to the airflow F circulating between the inlet 9 and outlet 10 of the sterilizing unit 1, so the air can thus be retained inside the domes 8 for longer a time period, thereby making the air oxidation process easier.

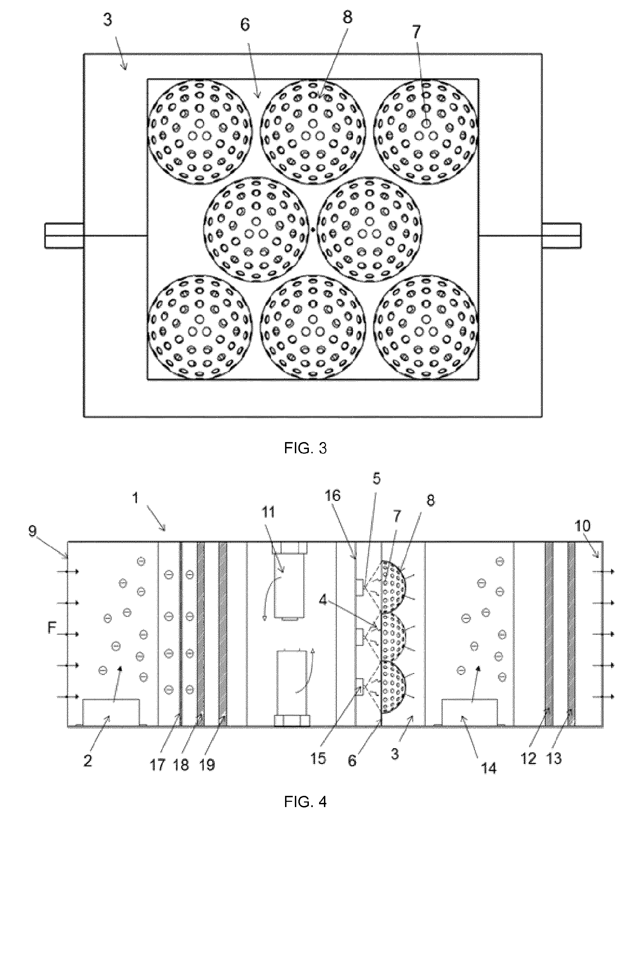

The photocatalytic oxidation chamber 3 of this first embodiment, shown in detail in Figure 3, comprises a plurality of domes 8 arranged in a plurality of rows. The domes 8 of each row are aligned with respect to their geometric center. Furthermore, the domes 8 in this embodiment are arranged in a staggered manner (quincunx), i.e., the domes 8 are arranged in parallel rows, such that the domes 8 of each row correspond to the middle region of the gaps of the intermediate row, such that they form equilateral triangles. In this embodiment, the domes 8 are arranged close to but without touching one another. The holes 7 of the domes 8 bring about an increase in air turbulence.

A second embodiment of the air sterilizing unit 1 shown in Figure 4 comprises ion generating means 2 in which air for photocatalytic oxidation is prepared, and a photocatalytic oxidation chamber 3 in which photocatalysis takes place. The features of the photocatalytic oxidation chamber 3 are similar to those of the first embodiment, so it is not considered necessary to describe them again.

The sterilizing unit 1 of the second embodiment further comprises ozone generating means 11 arranged between the ion generating means 2 and the photocatalytic oxidation chamber 3. Ozone is generated after the ions are generated since this order brings about plasma with greater oxidative capacity. Depending on the airflow F to be treated, the ozone generating means 11 can comprise one or several electronic devices based on a ceramic plate or tube. In other embodiments, ozone could be generated with other technologies known in the state of the art.

In this second embodiment, the sterilizing unit 1 further comprises filtering means 12 and 13 arranged after the photocatalytic oxidation chamber 3. The filtering means 12 and 13 comprise a catalyzing filter 12, for example, a copper oxide and manganese oxide filter, to absorb ozone. The filtering means 12 and 13 also comprise an activated carbon-based filter 12 for absorbing by-products generated during photocatalysis. In other embodiments, another type of filters can be used.

The sterilizing unit 1 further comprises second ion generating means 14 arranged between the photocatalytic oxidation chamber 3 and the filtering means 12 and 13. The objective of this second ionization is to negatively charge the air to improve subsequent filtering.

In this second embodiment, the sterilizing unit 1 further comprises a plurality of filters 17, 18 and 19 arranged between the ion generating means 2 and the ozone generating means 11. Said filters 17, 18 and 19 are arranged successively in the flow F direction in the following manner: a prefilter 17 is arranged first to collect larger particles; a Hepa-type filter 18 is arranged next to collect small PM2.5 and PM10 particles; and an activated carbon filter 19 is arranged last for absorbing volatile organic compounds and some harmful gases.

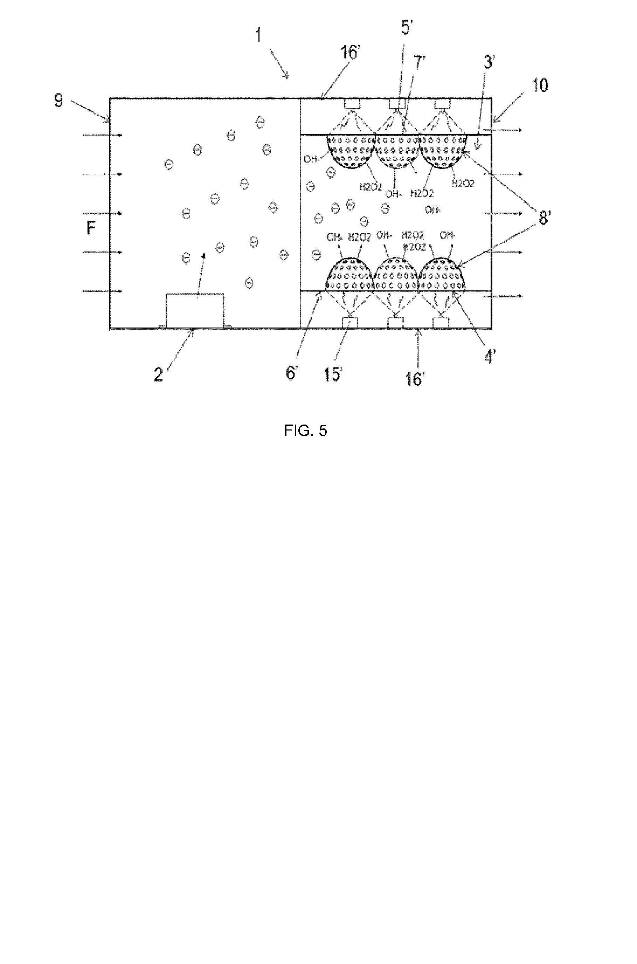

Figure 5 shows a third embodiment of the invention. This embodiment differs from the first embodiment in terms of the arrangement of the domes of the photocatalytic chamber. The rest of the features of the sterilizing unit 1 are similar to those of the first embodiment, so it is not considered necessary to describe them again.

In this third embodiment, the photocatalytic oxidation chamber 3' comprises two facing bases 6' between which the airflow F runs, said bases being parallel to the airflow F circulating between the inlet 9 and outlet 10 of the sterilizing unit 1, such that said airflow strikes the domes 8' laterally. The bases 6' comprise a plurality of respective domes 8' arranged facing one another. In this embodiment, the domes 8' arranged on the same base 6' are arranged close to but without touching one another. In other possible embodiments, the facing domes 8' can also be arranged close to but without touching one another.

Optionally, the outer surface of the domes 8' can be coated with titanium dioxide nanofibers. In this case, the closer the facing domes 8' are to one another, the greater the activation of the outer surfaces of the domes 8'.

In another embodiment (not shown in the drawings), the photocatalytic oxidation chamber comprises a plurality of domes which are arranged in a row, the domes being aligned with respect to their geometric center. Contiguous domes are arranged close to but without touching one another, the gap existing between the domes allowing air passage. The fact that the domes are so close to one another assures that most of the air mass going through the sterilizing unit is subjected to UV radiation. Furthermore, the holes of the domes bring about an increase in air turbulence. The base on which there are arranged domes can be arranged parallel or perpendicular to the airflow circulating between the inlet and outlet of the sterilizing unit.

Optionally, the sterilizing unit of the invention can comprise a fan (not shown in the drawings). Said fan can be arranged next to the inlet 9 or outlet 10 of the sterilizing unit 1. If the fan is arranged next to the outlet 10, it is a fan with an entraining effect. In contrast, if the fan is arranged next to the inlet 9, it is a fan with a push effect.

In the described embodiments, the titanium dioxide nanofibers with which the surfaces are coated to form the surfaces of activation 4 are preferably produced by means of an electrospinning process. Electrospinning is a known technique for producing nanofibers which consists of applying a potential difference between a material extrusion needle and a metal plate. The needle gradually discharges a solution consisting of dissolved nanoparticles in a polymeric carrier which, due to the potential difference between the needle and the plate; nanometric fibers are released and gradually superimposed on one another, creating a nanotissue.

Additionally, said nanofibers can be treated with a hydrothermal process. The specific surface area of the nanofibers can thus be increased.

The nanofibers produced by means of electrospinning have the drawback of not adhering well to non-planar surfaces. Since surfaces to be coated in this embodiment are dome-shaped, once the nanofibers are produced the surfaces are coated by means of a known process such as dip coating. This technique consists of submerging the part to be coated in a nanofiber dispersion with a solvent and then removing said part at a low and constant speed. The part is then baked to evaporate the solvent. The nanofibers are dispersed in the solvent without losing their structure and characteristics of interest.

In other embodiments, the nanofibers can be produced by means of other processes known by the person skilled in the art. For example, nanofibers can be synthesized with a hydrothermal process. Likewise, the surfaces can be coated using other known processes.

Optionally, in order to improve the photocatalytic capacity of titanium dioxide, the titanium dioxide nanofibers can be doped with silver and/or copper. Furthermore, the titanium dioxide nanofibers can also be doped with hydroxyapatite to increase and homogenize the moisture existing on the coated surface, thus improving photocatalysis.

## Claims

1. Air sterilizing unit comprising ion generating means (2) in which air for photocatalytic oxidation is prepared, and a photocatalytic oxidation chamber (3) in which photocatalysis takes place, **characterized in that** the photocatalytic oxidation chamber (3) comprises at least one surface of activation (4) coated with titanium dioxide nanofibers and arranged on a base (6), and at least one UV LED (5) irradiating light on said surface of activation (4), the shape of said surface of activation (4) being configured according to the radiation pattern of the LED (5).

2. Sterilizing unit according to the preceding claim, comprising at least one dome (8) with a plurality of holes (7), the LED (5) being located facing the inner surface of the dome (8), the inner surface of said dome (8) corresponding with the surface of activation (4).

3. Sterilizing unit according to the preceding claim, wherein the base (6) on which there is arranged said at least one dome (8) is arranged perpendicular to the airflow (F) circulating between the inlet (9) and outlet (10) of the sterilizing unit (1).

4. Sterilizing unit according to claim 2, wherein the base (6) on which there is arranged said at least one dome (8) is arranged parallel to the airflow (F) circulating between the inlet (9) and outlet (10) of the sterilizing unit (1), such that said airflow (F) strikes the domes (8) laterally.

5. Sterilizing unit according to claim 3 or 4, comprising a plurality of domes (8) which are arranged in at least one row, the domes (8) of each row preferably being aligned with respect to their geometric center.

6. Sterilizing unit according to the preceding claim, wherein there is a plurality of rows, the domes (8) being arranged in a staggered manner.

7. Sterilizing unit according to any of claims 3 to 6, comprising two facing bases (6) between which airflow (F) runs, said bases (6) being parallel to the airflow (F), and said bases (6) comprising respective domes (8) facing one another.

8. Sterilizing unit according to any of claims 2 to 7, wherein the domes (8) are half-spheres.

9. Sterilizing unit according to any of the preceding claims, wherein the titanium dioxide nanofibers with which the surface is coated to form the surface of activation (4) are produced by means of an electrospinning process, the titanium dioxide nanofibers preferably being treated with a hydrothermal process.

10. Sterilizing unit according to any of the preceding claims, wherein the surface of activation (4) is coated by means of a known process such as dip coating.

11. Sterilizing unit according to any of the preceding claims, wherein the titanium dioxide nanofibers are doped with silver and/or copper to increase the photocatalytic capacity of the titanium dioxide.

12. Sterilizing unit according to any of the preceding claims, wherein the titanium dioxide nanofibers are doped with hydroxyapatite to increase and homogenize the moisture existing on the surface of activation (4), thus improving photocatalysis.

13. Sterilizing unit according to any of the preceding claims, comprising ozone generating means (11) arranged between the ion generating means (2) and the photocatalytic oxidation chamber (3), and filtering means (12, 13) arranged after the photocatalytic oxidation chamber (3).

14. Sterilizing unit according to the preceding claim, wherein the filtering means (12, 13) comprise at least one copper oxide and manganese oxide filter (12) and an activated carbon-based filter (13).

15. Sterilizing unit according to any of the preceding claims, comprising second ion generating means (14) arranged between the photocatalytic oxidation chamber(3) and the filtering means (12, 13).
